# FASCICULE DE BREVET EUROPEEN

(11) **EP 4 703 011 B1**
(45) Date de publication et mention de la délivrance du brevet: **18.11.2026**
(21) Numéro de dépôt: 25186234.8
(22) Date de dépôt: 30.06.2025
(51) Int. Cl.: A61N 5/06

(54) **DISPOSITIF OPTIQUE DE STIMULATION ADAPTÉ POUR LE TRAITEMENT DE MALADIES, NOTAMMENT NEURODÉGÉNÉRATIVES**
OPTISCHE STIMULATIONSVORRICHTUNG ZUR BEHANDLUNG VON KRANKHEITEN, INSBESONDERE NEURODEGENERATIVEN ERKRANKUNGEN
OPTICAL STIMULATION DEVICE SUITABLE FOR TREATING DISEASES, IN PARTICULAR NEURODEGENERATIVE DISEASES

(30) Priorité: 30.08.2024 FR 2409268
(43) Date de publication de la demande: 04.03.2026
(73) Titulaire: Commissariat à l'Energie Atomique et aux Energies Alternatives, 75015 Paris (FR)
(72) Inventeur: BRUN-COSME-BRUNY, Marvin, 38054 GRENOBLE CEDEX 09 (FR); TORRES MARTINEZ, Napoléon, 38054 GRENOBLE CEDEX 09 (FR); BLEUET, Pierre, 38054 GRENOBLE CEDEX 09 (FR); ETAIX, Yvan, 38054 GRENOBLE CEDEX 09 (FR)
(74) Mandataire: INNOV-GROUP

(56) Documents cités:
- EP-B1- 3 834 885
- WO-A1-2022/197937
- US-A1- 2009 054 955
- US-A1- 2010 292 758
- US-A1- 2014 072 932
- US-A1- 2021 008 388
- US-A1- 2022 203 120
- US-A1- 2023 147 433
- US-A1- 2023 233 068

## Description

### Domaine technique de l'invention

L'invention vise un dispositif optique destiné à traiter ou ralentir l'évolution de maladies, notamment de maladies neurodégénératives.

### Etat de la technique

La photobiomodulation, également connue sous le nom de photothérapie, représente une modalité émergente qui vise à traiter ou à ralentir diverses pathologies au moyen de l'exposition à la lumière.

Dans ce contexte, la lumière englobe tout rayonnement électromagnétique, allant de l'ultraviolet à l'infrarouge lointain, en passant par le spectre visible. Les applications de la photobiomodulation s'étendent à un large éventail de domaines, englobant notamment les affections neurodégénératives, la fibromyalgie et la gestion de la douleur.

L'une des applications visées consiste à cibler la substance noire afin de ralentir la progression de la maladie de Parkinson. Actuellement, le protocole de suivi de la progression de la maladie implique que le patient se rende en clinique pour subir une irradiation afin d'imager la concentration de dopamine, biomarqueur du suivi de la maladie. Ce processus ionisant et contraignant est donc peu fréquent, mais primordial pour permettre au clinicien d'ajuster la stimulation (intensité lumineuse ou durée d'illumination) en fonction des résultats obtenus. Cet ajustement est réalisé de manière empirique et arbitraire par le clinicien.

Dans le brevet FR3126318B1, le dispositif de stimulation décrit comporte une source lumineuse et une unique fibre optique par laquelle le signal lumineux est émis et récupéré, via une solution optique de filtre sélectif.

Le brevet EP3834885B1 propose pour sa part de juxtaposer plusieurs modules le long de la sonde pour former une chaîne, chaque module comportant une source lumineuse et au moins un photodétecteur.

La série de demandes de brevets WO2019183075, WO2019183078, WO2019183054, WO2019183068 porte sur un système de stimulation optique doté d'une surveillance automatisée. Cette surveillance permet de faire en sorte que l'intensité du signal émis par la source lumineuse corresponde bien à ce qui est imposé par la consigne. Le but est une mesure de sécurité, afin de s'assurer que la diode (de type électroluminescente ou laser) vienne bien toujours émettre ce qui est imposé en consigne, et ainsi d'éviter les biais temporels dus à l'usure de l'électronique ou autres.

La demande de brevet US2021/008388A1 décrit pour sa part une sonde implantable et la demande de brevet WO2022/197937A1 concerne un dispositif de type NIR (Near Infra-Red).

Il reste nécessaire de pouvoir disposer d'un dispositif optique de stimulation des tissus qui puisse mesurer la lumière délivrée et déterminer le degré de dégénérescence des tissus pour adapter facilement le traitement effectué à l'évolution de la maladie, sans intervention du patient ou d'un professionnel de santé.

### Exposé de l'invention

Ce but est atteint par un dispositif optique de stimulation comportant :
- Une source lumineuse agencée pour produire un signal lumineux de stimulation,
- Un photodétecteur agencé pour capter un signal lumineux de réflexion,
- Des premiers moyens optiques couplés à ladite source lumineuse pour émettre ledit signal lumineux de stimulation et des deuxièmes moyens optiques couplés audit photodétecteur pour récupérer ledit signal lumineux de réflexion,
- Une unité de traitement et de commande connectée à ladite source lumineuse et audit photodétecteur, et configurée pour contrôler des données d'émission optique relatives au signal lumineux de stimulation produit par ladite source lumineuse et pour recevoir des données représentatives d'une réflexion optique en provenance dudit photodétecteur,
L'unité de traitement et de commande étant configurée pour :
- Acquérir plusieurs points de mesure à partir des données représentatives de la réflexion optique sur une fenêtre temporelle déterminée puis dériver lesdits points de mesure par rapport au temps,
- Déterminer une moyenne des points de mesure dérivés par rapport au temps,
- Exécuter une boucle de régulation consistant à comparer ladite moyenne déterminée à une valeur de consigne,
- Modifier au moins un paramètre de la boucle de régulation lorsque ladite moyenne reste de manière continue distincte de ladite valeur de consigne pendant une durée déterminée.

Selon une particularité, l'unité de traitement et de commande est configurée pour modifier ladite valeur de consigne lorsque ladite moyenne est égale à ladite valeur de consigne pendant une durée déterminée.

Selon une autre particularité, l'unité de traitement et de commande est configurée pour effectuer un lissage des points de mesure sur ladite fenêtre temporelle.

Selon une autre particularité, la grandeur représentative des données de réflexion optique acquises est un coefficient de réflexion optique.

Selon une autre particularité, les premiers moyens optiques comportent au moins une fibre optique émettrice couplée à la source lumineuse.

Selon une autre particularité, les premiers moyens optiques comportent au moins une fibre optique réceptrice couplée au photodétecteur.

Selon une autre particularité, le dispositif comporte un élément adaptateur recevant la fibre optique émettrice et la fibre optique réceptrice, ledit élément adaptateur étant configuré pour permettre un réglage en longueur de la fibre optique émettrice et/ou de la fibre optique réceptrice.

Selon une autre particularité, le dispositif comporte un bloc de résine positionné sur l'élément adaptateur pour figer la position de la fibre optique émettrice et de la fibre optique réceptrice.

Selon une autre particularité, la fibre optique émettrice et la fibre optique réceptrice comportent chacune une extrémité distale respectivement par rapport à la source lumineuse et au photodétecteur, et l'extrémité distale de la fibre optique réceptrice est en retrait par rapport à celle de la fibre optique émettrice.

L'invention consiste donc principalement à asservir automatiquement la stimulation optique des tissus cibles en utilisant une boucle-fermée entre la stimulation optique et le suivi de l'évolution du tissu ciblé impliqué dans la maladie.

### Brève description des figures

D'autres caractéristiques et avantages vont apparaître dans la description détaillée qui suit faite en regard des dessins annexés dans lesquels :
- La figure 1A représente de manière schématique le dispositif optique de stimulation conforme à l'invention ;
- La figure 1B représente une variante de réalisation du dispositif optique de stimulation ;
- La figure 2 représente le schéma de la boucle de régulation mise en œuvre lors de l'utilisation du dispositif optique de stimulation de l'invention ;
- La figure 3 illustre le principe d'acquisition des points de mesure à l'aide du photodétecteur ;

### Description détaillée d'au moins un mode de réalisation

Dans la suite de la description, les termes "proximal" et "distal" sont à comprendre en tenant compte du sens d'émission des signaux optiques de la source vers l'extrémité du dispositif.

L'invention concerne plus particulièrement un dispositif optique de stimulation destiné à illuminer des tissus T.

Ce dispositif permet notamment de réaliser une illumination localisée (par exemple dans le proche infra-rouge ou avec toute autre longueur d'onde selon le traitement envisagé - traitement type neuroprotection, optogénétique) des tissus cibles (par exemple SNc, hippocampe, striatum...). Ce dispositif peut servir en particulier dans le traitement de maladies neurodégénératives telles que la maladie de Parkinson, Alzheimer, Huntington...

L'illumination des tissus T peut avoir différents objectifs selon l'application : neuroprotection, optogénétique, stimulation... Plusieurs cibles sont concernées, par exemple la substance noire compacta (SNc) qui dégénère dans la maladie de Parkinson, l'hippocampe, principal noyau mis en cause dans la maladie d'Alzheimer, ou le striatum pour la maladie d'Huntington. L'illumination peut être apportée directement dans les tissus (avec risque de lésions supplémentaires) ou en choisissant des trajectoires passant par les ventricules (cavités permettant la circulation du liquide céphalorachidien LCR) et en contact ou à proximité des structures à traiter (illuminateur directifs).

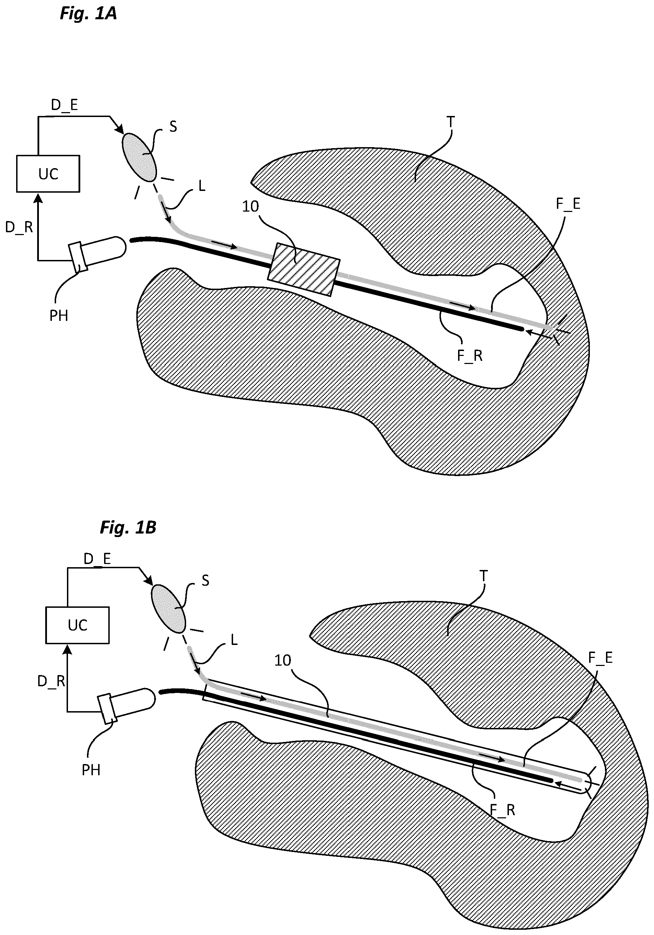

En référence à la figure 1A et à la figure 1B, le dispositif de l'invention comporte un module de commande comprenant un boîtier intégrant une unité de traitement et de commande UC. Cette unité de traitement et de commande UC comporte avantageusement un générateur d'impulsions implantable (appelé couramment IPG pour "Implantable Pulse Generator"). De manière connue, un générateur d'impulsions implantable de type IPG comporte principalement une carte électronique et une batterie, rechargeable ou non. La carte électronique comporte un microcontrôleur chargé de gérer le fonctionnement du générateur. Le générateur comporte avantageusement plusieurs canaux de connexion.

Le dispositif comporte également une source lumineuse S contrôlée par le générateur d'impulsions implantable. Cette source lumineuse S peut être intégrée au module de commande. La source lumineuse S peut être composée par exemple d'une ou plusieurs diodes électroluminescentes ou diode Laser. Elle est choisie (éventuellement avec des moyens de filtrage adaptés) pour émettre un signal lumineux L à la longueur d'onde choisie, selon le traitement/suivi à réaliser. Le signal lumineux L est par exemple émis à une intensité désignée l_lum pendant une durée d'illumination déterminée et choisie. Le dispositif comporte des premiers moyens optiques couplés à ladite source lumineuse S pour émettre ledit signal lumineux L de stimulation.

Ces premiers moyens optiques comportent avantageusement au moins une première fibre optique F_E, dite émettrice, employée pour la stimulation.

Le dispositif comporte un photodétecteur PH, par exemple également intégré au module de commande.

Le dispositif comporte des deuxièmes moyens optiques couplés au photodétecteur PH pour récupérer le signal optique de réflexion issu de la réflexion des tissus, après stimulation, et le guider vers le photodétecteur PH.

Ces deuxièmes moyens optiques comportent avantageusement au moins une deuxième fibre optique F_R, dite réceptrice ou de retour.

De manière non limitative, la fibre optique émettrice F_E peut être implantée par son extrémité distale, en profondeur, dans le tissu cible sur une distance pouvant aller de 1mm à 5mm de la surface des tissus, cette profondeur d'enfoncement dépendant de l'épaisseur et de la réflectance du tissu. Elle doit être choisie pour que la lumière soit bien diffusée au sein du tissu et réfléchie.

Dans le cadre de l'invention, l'extrémité distale de la fibre optique réceptrice F_R reste en retrait par rapport à la surface du tissu T pour y capter la réflexion optique qui en émane.

Dans un mode de réalisation particulier représenté sur la figure 1A, il est possible de prévoir un élément adaptateur 10 d'assemblage, permettant de réunir les deux fibres optiques F_E, F_R. Cet élément adaptateur 10 permet par ailleurs de régler la position longitudinale de chaque fibre et donc le décalage en longueur entre les extrémités distales des deux fibres. La position longitudinale de chaque fibre optique peut être réglée manuellement, puis fixée en employant une résine.

Cet élément adaptateur 10 peut présenter une première traversée destinée au passage de la fibre optique émettrice, avec un diamètre équivalent ou supérieur à celui de ladite fibre (gaine comprise). L'élément adaptateur 10 peut comporter une deuxième traversée destinée au passage de la fibre optique réceptrice F_R, ayant un diamètre inférieur à celui de la fibre (avec gaine), mais supérieur à celui de cette même fibre sans gaine. Ainsi, en dénudant le bout de la fibre optique réceptrice F_R de sa gaine sur moins de 1 mm depuis son extrémité distale, la fibre ne peut dépasser la base de l'élément adaptateur 10 que sur cette longueur de dénudement. Le tout est fixé par une résine introduite au sein des orifices de l'élément adaptateur 10. L'élément adaptateur 10 est réalisé à l'aide d'un matériau biocompatible le plus réfléchissant possible tel que le titane, éventuellement recouvert de silicone.

En liaison avec la figure 1B, il faut noter qu'il est également possible de réunir les deux fibres optiques au sein d'une même sonde 100 se présentant sous la forme d'une tige allongée souple, suivant une direction longitudinale. Les deux fibres optiques sont par exemple identiques, arrangées en parallèle au sein de la sonde. Les deux fibres sont par exemple enrobées dans un matériau de type silicone ou équivalent présentant les caractéristiques adaptées à l'application. L'extrémité distale de la sonde 100 peut présenter une forme atraumatique. Un décalage en longueur entre les extrémités distales des deux fibres F_E, F_R peut alors être figé par l'enrobage.

L'unité de traitement et de commande UC comporte un microprocesseur capable d'exécuter une séquence comportant des phases de stimulation et des phases de surveillance, via la commande de la source lumineuse S et la réception de données par le photodétecteur PH. Une mémoire reliée au microprocesseur permet l'enregistrement des données de réflexion optique captées par le photodétecteur PH. L'unité de traitement et de commande UC est configurée pour contrôler la source lumineuse S et pour acquérir des données en provenance du photodétecteur PH.

L'invention consiste notamment à mettre en place une boucle fermée de régulation entre la stimulation optique du tissu T et le suivi in situ de l'évolution du tissu T. L'invention s'appuie notamment sur le fait que les propriétés optiques des tissus (notamment en réflexion) sont susceptibles d'évoluer dans le temps selon l'avancée de la maladie chez le patient. L'invention permet ainsi de détecter l'état d'avancement de la maladie en récupérant la lumière réfléchie sur des tissus T, marqueur de l'évolution de la maladie, dans l'objectif d'établir une boucle fermée sur la stimulation asservie au suivi.

L'unité de traitement et de commande UC est configurée pour exécuter cette boucle fermée de régulation entre les données D_R représentatives de la réflexion optique en provenance du photodétecteur PH et les données D_E d'émission optique relatives au signal lumineux L de stimulation produit par la source lumineuse S. Les données D_E d'émission optique peuvent comporter un ou plusieurs paramètres choisis parmi l'intensité l_lum du signal lumineux L, la durée d'émission, le rapport cyclique...

Les données D_R représentatives de la réflexion optique acquises par le photodétecteur PH sont enregistrées dans la mémoire à une fréquence d'acquisition définie.

Le dispositif médical étant destiné à être implanté à vie, les données sont vouées à un enregistrement perpétuel sans interruption. La boucle de rétroaction asservissant la stimulation optique s'appuie sur ces données enregistrées et est avantageusement de type PID (proportionnelle-Intégrale-dérivée).

L'unité de traitement et de commande UC définit une fenêtre temporelle correspondant à N points de mesure de données D_R représentatives de la réflexion optique (N supérieur ou égal à 2, N étant avantageusement supérieur à 10, avantageusement au moins égal à 100). Chaque point de mesure est généré à un pas de temps déterminé selon la fréquence d'acquisition (par exemple toutes les heures, ou même toutes les 6h ou toutes les 12h).

L'unité de traitement et de commande UC traite les N points de mesure sur ladite fenêtre temporelle et calcule une moyenne M_i sur les points de mesure traités.

L'unité de traitement et de commande UC vient comparer ladite moyenne M_i déterminée à une valeur de consigne C prédéfinie.

L'unité de traitement et de commande UC peut alors effectuer différentes actions, selon le résultat de la comparaison.

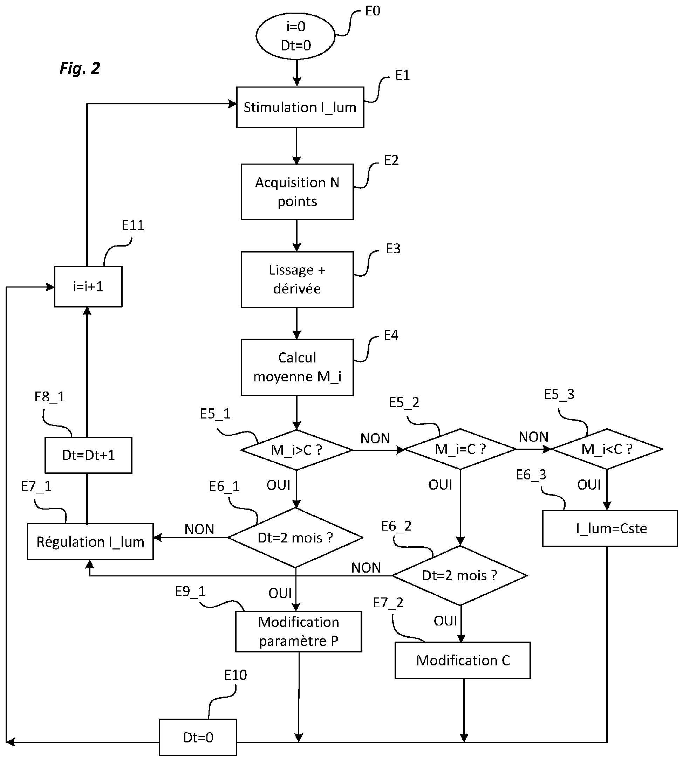

L'unité de traitement et de commande UC a notamment la capacité de modifier au moins un paramètre (P, I et/ou D) relatif au régulateur employé dans la boucle de régulation, ou la valeur de consigne C, selon différentes situations de fonctionnement décrites ci-dessous en liaison avec la figure 2.

Les points de mesure sont par exemple des points acquis par le photodétecteur PH, ces points étant représentatifs du coefficient de réflexion optique.

Le traitement effectué sur les points de mesure peut avantageusement consister en un lissage, pour éliminer le bruit.

Le lissage est par exemple réalisé en utilisant un filtre passe-bas de type gaussien, médian ou autre. Ce lissage permet de réduire le bruit de fluctuation temporelle lié à l'acquisition.

Le traitement effectué sur les points de mesure consiste également en une dérivée par rapport au temps de chaque point de mesure, obtenu après lissage.

Il faut noter qu'il est particulièrement pertinent de travailler sur une variation temporelle et non sur une donnée pure obtenue en sortie du photodétecteur PH. Le coefficient de réflexion optique est relié à l'intensité électrique délivrée par le photodétecteur PH. Cependant, il est plus pertinent d'étudier la dérivée en temps de cette intensité électrique, qui équivaut à la dérivée temporelle du coefficient de réflexion optique, pondéré d'un gain lié à l'électronique. Il faut noter que les tissus T ne pourront jamais se régénérer par stimulation lumineuse (autrement dit par photobiomodulation). Les cellules mortes restent mortes, même après stimulation. Le coefficient de réflexion optique ne sera jamais en diminution : Dans le meilleur des cas, il pourra se stabiliser. La boucle de régulation employée dans l'invention permet d'imposer une valeur de consigne sur une variation temporelle. Ce principe s'applique à tout tissu T bénéficiant d'un traitement contre une maladie aux séquelles irréversibles sur les tissus.

La moyenne M_i correspond ainsi par exemple à une vitesse de réflexion (par exemple exprimée par mois) et est représentative de la vitesse de progression de la pathologie.

La fenêtre temporelle étant glissante, une nouvelle moyenne M_i est calculée sur les N derniers points de mesure lissés et dérivés, pour chaque nouveau point de mesure.

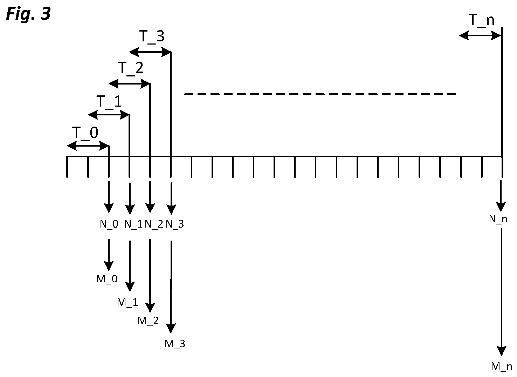

La figure 3 permet d'illustrer le principe d'acquisition. Au bout d'une durée T_0, l'unité de traitement et de commande UC a acquis N_0 points de mesure et calcule la moyenne M_0 selon les principes décrits ci-dessus.

A chaque nouveau point de mesure ou ensemble de points, l'unité de traitement et de commande s'appuie sur les N_1 derniers points de mesure, acquis sur la période T_1 et calcule, après lissage et dérivation, la moyenne M_1 sur les N_1 points de mesure. Et ainsi de suite pour chaque nouveau point de mesure ou ensemble de points. L'unité de traitement a acquis N_2 points de mesure sur la période T_2 et calcule la moyenne M_2 sur ces N_2 points de mesure lissés et dérivés et acquis N_3 points de mesure sur la période T_3 pour déterminer la moyenne M_3 sur les N_3 points de mesure lissés et dérivés. Les périodes T_0, T_1, T_2 et T_3 sont identiques. Le principe se poursuit pour chaque nouvelle période jusqu'à la période T_n avec les derniers points de mesure N_n et la moyenne M_n.

L'unité de de traitement et de commande UC compare chaque moyenne M_i calculée à la valeur de consigne C, définissant une vitesse de réflexion maximale à ne pas dépasser. Cette valeur de consigne C est choisie de manière arbitraire et peut être ajustée par l'unité de traitement et de commande UC. Dans le présent exemple, cette valeur de consigne C est fixée à 0.05 mois-1.

La figure 2 montre un exemple de réalisation d'un algorithme exécuté par l'unité de traitement et de commande UC.

Dans cet exemple, la boucle de régulation est mise en œuvre sur l'intensité l_lum du signal lumineux L de stimulation mais il faut noter qu'il serait possible de jouer sur d'autres paramètres, notamment la durée de stimulation, ainsi que sur la modulation et le rapport cyclique du signal lumineux L émis. Ces différents paramètres peuvent être considérés de manière individuelle dans la boucle de régulation, ou gérés de manière cumulative. Un algorithme peut être mis en œuvre pour adapter chacun de ces paramètres, selon la régulation à effectuer.

E0 : Il s'agit d'une étape d'initialisation à 0 des deux paramètres i et Dt, utilisés dans la mise en œuvre de la boucle. i correspond à un indice d'itération et Dt correspond à une durée.

E1 : Sur la base de la boucle de régulation, le dispositif stimule les tissus T en fournissant un signal lumineux de stimulation à l'intensité l_lum.

E2 : Au fur et à mesure de la stimulation, l'unité de traitement et de commande UC acquière des données de réflexion optique D_R. L'unité de traitement et de commande acquière N points de mesure, N étant supérieur ou égal à 2. Il s'agit par exemple des 100 derniers points. Elle applique ainsi le principe décrit ci-dessus en liaison avec la figure 3.

E3 : Sur les N derniers points acquis, l'unité de traitement et de commande UC effectue avantageusement un lissage de la courbe obtenue et calcule la dérivée de la fonction correspondante.

E4 : L'unité de traitement et de commande UC détermine une moyenne M_i sur l'ensemble des N points lissés et dérivés. M_i correspond à la moyenne sur les N derniers points pour l'itération i.

E5_1 : L'unité de traitement et de commande effectue un premier test sur la moyenne M_i en la comparant à la valeur de consigne C. Ce premier test consiste à vérifier si la moyenne M_i est supérieure à la valeur de consigne C. La valeur de consigne C est une valeur fixe et pré-mémorisée.

E6_1 : Si M_i est supérieure à la valeur de consigne C, l'unité de traitement et de commande UC effectue un test pour déterminer si cette inégalité perdure depuis une durée déterminée. Pour cela, l'unité de traitement et de commande regarde si le compteur de temps Dt est supérieur à cette durée, par exemple fixée à 2 mois. Cette durée est choisie de manière arbitraire. Elle pourrait être plus longue ou plus courte. E7_1 : Tant que la durée Dt n'est pas dépassée, la régulation se poursuit normalement et l'unité de traitement et de commande UC exécute la boucle de régulation en stimulant les tissus avec l'intensité l_lum. Dans cette boucle de régulation, l'unité de traitement et de commande UC détermine l'intensité du signal lumineux de stimulation l_lum. Cette intensité est modifiée en l_lum + P(M_i-C), le terme P(M_i-C) définissant la correction appliquée à chaque itération de la boucle, P étant le paramètre du régulateur.

E8_1 : Tant que la durée Dt n'est pas dépassée, l'unité de traitement et de commande UC incrémente le compteur de temps Dt.

E11 : Puis, l'unité de traitement et de commande UC incrémente ensuite l'itération i. L'algorithme reprend ensuite à l'étape E1 décrite ci-dessus.

E9_1 : Si M_i est restée supérieure à la valeur de consigne pendant toute la durée Dt, l'unité de traitement et de commande UC vient modifier le paramètre P utilisé dans le régulateur. Le paramètre P du régulateur est par exemple modifié en P(1±b), avec b un paramètre d'incrémentation/décrémentation non nul. L'unité de traitement et de commande UC vient ainsi corriger la boucle de régulation et son fonctionnement.

E5_2 : Si M_i n'est pas supérieure à la valeur de consigne C, l'unité de traitement et de commande effectue un test pour voir si M_i est égale à la valeur de consigne C (avec une certaine tolérance, par exemple avec un écart fixé à +/-5% maximum).

E6_2 : Si M_i est égale à la valeur de consigne C, l'unité de traitement et de commande UC effectue un test pour déterminer si cette égalité perdure depuis une durée déterminée. Pour cela, l'unité de traitement et de commande UC regarde si le compteur de temps Dt est supérieur à cette durée, par exemple fixée à 2 mois. Cette durée est choisie de manière arbitraire. Elle pourrait être plus longue ou plus courte. L'algorithme est exécuté de manière similaire au cas M_i>C (étapes E7_1 et E8_1).

E7_1 : Tant que la durée Dt n'est pas dépassée, la régulation se poursuit normalement et l'unité de traitement et de commande UC exécute la boucle de régulation en stimulant les tissus avec l'intensité l_lum. Dans cette boucle de régulation, l'unité de traitement et de commande détermine l'intensité du signal lumineux de stimulation l_lum. Cette intensité est modifiée en l_lum + P(M_i-C), le terme P(M_i-C) définissant la correction appliquée à chaque itération de la boucle, P étant le paramètre du régulateur.

E8_1 : Tant que la durée Dt n'est pas dépassée, l'unité de traitement et de commande UC incrémente le compteur de temps Dt.

E11 : Puis, l'unité de traitement et de commande UC incrémente ensuite l'itération i. L'algorithme reprend ensuite à l'étape E1 décrite ci-dessus.

E7_2 : Si M_i est égale à la valeur de consigne C durant la durée Dt, l'unité de traitement et de commande vient modifier la valeur de consigne C. La valeur de consigne C est par exemple modifiée en C(1-a), a étant également un paramètre non nul d'incrémentation/décrémentation.

E5_3 : Si M_i n'est pas supérieure à la valeur de consigne C, l'unité de traitement et de commande UC effectue un test pour voir si M_i est inférieure à la valeur de consigne C. E6_3 : Si M_i est inférieure à la valeur de consigne C, l'unité de traitement et de commande UC maintient l'intensité l_lum du signal lumineux de stimulation à une valeur constante.

E10 : Après les étapes E9_1, E7_2 et E6_3, l'unité de traitement et de commande UC remet le compteur de temps Dt à 0. Dans ce cas, elle repart pour une nouvelle durée Dt.

E11 : Après l'étape E10, l'unité de traitement et de commande UC incrémente l'itération i. L'algorithme reprend ensuite à l'étape E1 décrite ci-dessus.

Dans le cas du traitement de la maladie de Parkinson et de la surveillance de son évolution, le but est de faire en sorte que la source lumineuse S émette un signal lumineux de stimulation avec une intensité l_lum constante à destination de la substance noire, et d'analyser la variation du coefficient de réflexion au cours du temps.

L'analyse effectuée prend en compte la progression temporelle et relative du signal réfléchie. Les systèmes à évolution lente et à évolution rapide peuvent tous deux être pris en compte par cette boucle fermée, notamment par l'étude de la dérivée temporelle de la réflexion optique selon différents pas de temps.

Comme indiqué ci-dessus, il est possible de jouer sur un ou plusieurs autres paramètres que l'intensité l_lum du signal lumineux de stimulation.

Il faut noter que l'incrémentation/décrémentation de la valeur de consigne C et du paramètre P par a et b sont généralisables à toutes fonctions croissantes ou décroissantes réelles.

De même, l'invention est décrite ci-dessus sur la base du paramètre proportionnel P de la boucle PID. Mais ce paramètre P fait partie des trois paramètres P, I et D, I étant le paramètre intégratif et D le paramètre dérivatif. De manière générale la correction appliquée par le régulateur peut être écrite : $P * \left(M_i-C\right) + I * integrale \left(M_i-C,0,ti\right) + D * derivée \left(M_i-C\right)$

Il serait donc envisageable de venir modifier les autres paramètres I et D de la boucle de régulation lors de l'étape E9_1 décrite ci-dessus.

Afin d'étudier les effets bénéfiques de ce type de boucle fermée, une simulation numérique de mort cellulaire d'un tissu atteint par la maladie de Parkinson a été réalisée. A chaque pas de temps, on a considéré l'état d'une cellule (morte ou vivante) au moyen de deux tirages selon des distributions gaussiennes. Il faut noter que la mort cellulaire se traduit par une réflexion optique croissante. Par conséquent, à une donnée de mort cumulée est ajouté du bruit blanc simulant l'acquisition des données de réflexion optique.

De manière concrète, le dispositif présente par exemple les particularités suivantes :
- Une pile rechargeable de 3,7 V est employée pour fournir du courant à une diode laser (référence Ushio HL6756MG) émettant à une longueur d'onde de 670 nm, jusqu'à 15 mW de puissance optique. Le courant fourni atteint environ 45 mA.
- Au sein d'un boîtier, la diode laser est couplée à une lentille asphérique (référence LightPath #3555200) focalisatrice communiquant sa lumière à une fibre optique (émission) d'ouverture numérique N.A. = 0.37 (référence OFS #HCP-M0200T). L'extrémité distale de la fibre hors du boîtier est implantée sur 1 à 3 mm dans le tissu cible, où la lumière diffuse et est réfléchie.

- Une fibre optique parallèle d'ouverture numérique N.A. = 0.57 (référence IDIL #OPFIB02968), en retrait par rapport à la première et en dehors du tissu, permet une grande capture des photons réfléchis par le tissu, et leur transmission à l'extrémité proximale de la fibre optique dans le boîtier.
- La lumière en sortie est collimatée par une lentille asphérique (référence AMS Technologies #LD1560) sur une photodiode (référence Osram #SFH2200). Spatialement, cette photodiode est disposée en parallèle de la diode laser sur le circuit imprimé, ayant une sensibilité de 0.5 A/W à 670 nm de longueur d'onde, et une surface active de 7.02 mm².
- Chacune des deux fibres optiques est enveloppée d'une gaine et de silicone opacifié (référence NuSil Technology #MED-6233 et #MED-4800-2), évitant la communication directe des photons émis sur la fibre réceptrice. Les deux gaines sont collées en parallèle par une résine époxy (référence EPOTEK #301), le bout de la fibre émettrice dépasse de 2 mm ou plus celui de la fibre réceptrice au moyen d'un adaptateur de longueur (voir ci-dessus). Ainsi les photons émis sont d'abord contraints de se disperser et se réfléchir dans le tissu ciblé avant d'être captés par la fibre réceptrice.
- Au sein du boîtier, un microcontrôleur (référence STM32 #L011D3P6) contrôle les cycles d'alimentation de la diode laser et d'acquisition de la photodiode, l'enregistrement des données sur une mémoire EEprom (référence Microchip Technology #AT24CM02) et leur traitement. Une programmation du microcontrôleur permet d'établir une modulation de la puissance de lumière émise en fonction de la lumière réfléchie captée par la photodiode, par boucle-fermée auto-entretenue avec retour de l'état.

La photodiode d'analyse doit pouvoir capter les photons réfléchis et transmis par la fibre optique réceptrice F_R, et ce tout en demeurant dans un régime linéaire, c'est-à-dire où le flux lumineux reçu par la photodiode est proportionnel à l'intensité du courant qu'elle génère. Un flux lumineux capté par la photodiode fait varier l'intensité de courant traversant la résistance adjacente, et donc sa tension. Il en résulte qu'un flux lumineux trop fort ou trop faible risquerait d'augmenter la tension aux bornes de la photodiode endehors de son régime linéaire.

La solution de l'invention présente ainsi de nombreux avantages, parmi lesquels :
- Elle permet une régulation de l'intensité ou d'autres paramètres du signal lumineux L de stimulation, tenant compte de l'évolution de la maladie ;
- L'algorithme mis en œuvre permet de venir corriger automatiquement les paramètres de la boucle de régulation et éventuellement la valeur de consigne, en fonction de l'évolution de la maladie, permettant une auto-adaptation.
- Le dispositif peut rester à demeure sur une très longue période.
- Il permet une adaptation en longueur des deux fibres optiques du dispositif.

## Revendications

1. Dispositif optique de stimulation comportant :
- Une source lumineuse (S) agencée pour produire un signal lumineux (L) de stimulation,
- Un photodétecteur (PH) agencé pour capter un signal lumineux de réflexion,
- Des premiers moyens optiques couplés à ladite source lumineuse (S) pour émettre ledit signal lumineux (L) de stimulation et des deuxièmes moyens optiques couplés audit photodétecteur (PH) pour récupérer ledit signal lumineux de réflexion,
- Une unité de traitement et de commande (UC) connectée à ladite source lumineuse (S) et audit photodétecteur (PH), et configurée pour contrôler des données (D_E) d'émission optique relatives au signal lumineux (L) de stimulation produit par ladite source lumineuse (S) et pour recevoir des données (D_R) représentatives d'une réflexion optique en provenance dudit photodétecteur (PH),
**Caractérisé en ce que** l'unité de traitement et de commande (UC) est configurée pour :
- Acquérir plusieurs points de mesure à partir des données (D_R) représentatives de la réflexion optique sur une fenêtre temporelle déterminée puis dériver lesdits points de mesure par rapport au temps,
- Déterminer une moyenne (M_i) des points de mesure dérivés par rapport au temps,
- Exécuter une boucle de régulation consistant à comparer ladite moyenne déterminée à une valeur de consigne (C),
- Modifier au moins un paramètre de la boucle de régulation lorsque ladite moyenne (M_i) reste de manière continue distincte de ladite valeur de consigne (C) pendant une durée (Dt) déterminée.

2. Dispositif selon la revendication 1, **caractérisé en ce que** l'unité de traitement et de commande (UC) est configurée pour modifier ladite valeur de consigne (C) lorsque ladite moyenne (M_i) est égale à ladite valeur de consigne (C) pendant une durée (Dt) déterminée.

3. Dispositif selon la revendication 1 ou 2, **caractérisé en ce que** l'unité de traitement et de commande (UC) est configurée pour effectuer un lissage des points de mesure sur ladite fenêtre temporelle.

4. Dispositif selon l'une des revendications 1 à 3, **caractérisé en ce que** la grandeur représentative des données de réflexion optique acquises est un coefficient de réflexion optique.

5. Dispositif selon l'une des revendications 1 à 4, **caractérisé en ce que** les premiers moyens optiques comportent au moins une fibre optique émettrice (F_E) couplée à la source lumineuse (S).

6. Dispositif selon l'une des revendications 1 à 5, **caractérisé en ce que** les premiers moyens optiques comportent au moins une fibre optique réceptrice (F_R) couplée au photodétecteur (PH).

7. Dispositif selon la revendication 6, **caractérisé en ce qu'**il comporte un élément adaptateur (10) recevant la fibre optique émettrice (F_E) et la fibre optique réceptrice (F_R), ledit élément adaptateur (10) étant configuré pour permettre un réglage en longueur de la fibre optique émettrice et/ou de la fibre optique réceptrice.

8. Dispositif selon la revendication 7, **caractérisé en ce qu'**il comporte un bloc de résine positionné sur l'élément adaptateur pour figer la position de la fibre optique émettrice et de la fibre optique réceptrice.

9. Dispositif selon la revendication 7 ou 8, **caractérisé en ce que** la fibre optique émettrice (F_E) et la fibre optique réceptrice (F_R) comportent chacune une extrémité distale respectivement par rapport à la source lumineuse (S) et au photodétecteur (PH), et **en ce que** l'extrémité distale de la fibre optique réceptrice (F_R) est en retrait par rapport à celle de la fibre optique émettrice (F_E).

## Patentansprüche

1. Optische Stimulationsvorrichtung, umfassend:
- eine Lichtquelle (S), die dazu ausgelegt ist, ein Stimulationslichtsignal (L) zu erzeugen,
- einen Photodetektor (PH), der dazu ausgelegt ist, ein Reflexionslichtsignals aufzunehmen,
- erste optische Mittel, die mit der Lichtquelle (S) gekoppelt sind, um das Stimulationslichtsignal (L) zu emittieren, und zweite optische Mittel, die mit dem Photodetektor (PH) gekoppelt sind, um das Reflexionslichtsignal zurückzugewinnen,
- eine Verarbeitungs- und Steuereinheit (UC), die mit der Lichtquelle (S) und mit dem Photodetektor (PH) verbunden ist und dazu ausgebildet ist, optische Emissionsdaten (D_E) bezüglich des von der Lichtquelle (S) erzeugten Stimulationslichtsignals (L) zu überwachen und Daten (D_R), die für eine von dem Photodetektor (PH) kommende optische Reflexion repräsentativ sind, zu empfangen,
**dadurch gekennzeichnet, dass** die Verarbeitungs- und Steuereinheit (CPU) dazu ausgebildet ist:
- ausgehend von den Daten (D_R), die für die optische Reflexion repräsentativ sind, über ein bestimmtes Zeitfenster mehrere Messpunkte zu erfassen und dann die Messpunkte in Bezug auf die Zeit abzuleiten,
- einen Mittelwert (M_i) der in Bezug auf die Zeit abgeleiteten Messpunkte zu bestimmen,
- eine Regelschleife auszuführen, die darin besteht, den bestimmten Mittelwert mit einem Sollwert (C) zu vergleichen,
- mindestens einen Parameter der Regelschleife zu ändern, wenn der Mittelwert (M_i) während einer bestimmten Dauer (Dt) kontinuierlich von dem Sollwert (C) verschieden bleibt.

2. Vorrichtung nach Anspruch 1, **dadurch gekennzeichnet, dass** die Verarbeitungs- und Steuereinheit (UC) dazu ausgebildet ist, den Sollwert (C) zu ändern, wenn der Mittelwert (M_i) während einer bestimmten Dauer (Dt) gleich dem Sollwert (C) ist.

3. Vorrichtung nach Anspruch 1 oder 2, **dadurch gekennzeichnet, dass** die Verarbeitungs- und Steuereinheit (UC) dazu ausgebildet ist, eine Glättung der Messpunkte über das Zeitfenster durchzuführen.

4. Vorrichtung nach einem der Ansprüche 1 bis 3, **dadurch gekennzeichnet, dass** die für die erfassten optischen Reflexionsdaten repräsentative Größe ein optischer Reflexionskoeffizient ist.

5. Vorrichtung nach einem der Ansprüche 1 bis 4, **dadurch gekennzeichnet, dass** die ersten optischen Mittel mindestens eine emittierende optische Faser (F_E) umfassen, die mit der Lichtquelle (S) gekoppelt ist.

6. Vorrichtung nach einem der Ansprüche 1 bis 5, **dadurch gekennzeichnet, dass** die ersten optischen Mittel mindestens eine empfangende optische Faser (F_R) umfassen, die mit dem Photodetektor (PH) gekoppelt ist.

7. Vorrichtung nach Anspruch 6, **dadurch gekennzeichnet, dass** sie ein Adapterelement (10) umfasst, das die emittierende optische Faser (F_E) und die empfangende optische Faser (F_R) aufnimmt, wobei das Adapterelement (10) dazu ausgebildet ist, eine Längeneinstellung der emittierenden optischen Faser und/oder der empfangenden optischen Faser zu ermöglichen.

8. Vorrichtung nach Anspruch 7, **dadurch gekennzeichnet, dass** sie einen Harzblock umfasst, der auf dem Adapterelement positioniert ist, um die Position der emittierenden optischen Faser und der empfangenden optischen Faser zu fixieren.

9. Vorrichtung nach Anspruch 7 oder 8, **dadurch gekennzeichnet, dass** die emittierende optische Faser (F_E) und die empfangende optische Faser (F_R) jeweils ein in Bezug auf die Lichtquelle (S) bzw. den Photodetektor (PH) distales Ende aufweisen und dass das distale Ende der empfangenden optischen Faser (F_R) in Bezug auf das der emittierenden optischen Faser (F_E) zurückversetzt ist.

## Claims

1. Optical stimulation device comprising:
- A light source (S) arranged to produce a stimulation light signal (L),
- A photodetector (PH) arranged to capture a reflection light signal,
- First optical means coupled to said light source (S) for emitting said stimulation light signal (L), and second optical means coupled to said photodetector (PH) for recovering said reflection light signal,
- A processing and control unit (UC) connected to said light source (S) and to said photodetector (PH), and configured to monitor optical emission data (D_E) relating to the stimulation light signal (L) produced by said light source (S) and to receive data (D_R) which are representative of an optical reflection originating from said photodetector (PH),
**Characterized in that** the processing and control unit (UC) is configured to:
- Acquire multiple measurement points from the data (D_R) which are representative of the optical reflection over a determined time window and then derive said measurement points with respect to time,
- Determine an average (M_i) of the measurement points derived with respect to time,
- Execute a control loop consisting in comparing said determined average with a setpoint value (C),
- Modify at least one parameter of the control loop when said average (M_i) remains continuously distinct from said setpoint value (C) for a determined duration (Dt).

2. Device according to Claim 1, **characterized in that** the processing and control unit (UC) is configured to modify said setpoint value (C) when said average (M_i) is equal to said setpoint value (C) for a determined duration (Dt).

3. Device according to Claim 1 or 2, **characterized in that** the processing and control unit (UC) is configured to smooth the measurement points over said time window.

4. Device according to one of Claims 1 to 3, **characterized in that** the quantity which is representative of the acquired optical reflection data is an optical reflection coefficient.

5. Device according to one of Claims 1 to 4, **characterized in that** the first optical means comprise at least one emitting optical fibre (F_E) coupled to the light source (S).

6. Device according to one of Claims 1 to 5, **characterized in that** the first optical means comprise at least one receiving optical fibre (F_R) coupled to the photodetector (PH).

7. Device according to Claim 6, **characterized in that** it comprises an adapting element (10) receiving the emitting optical fibre (F_E) and the receiving optical fibre (F_R), said adapting element (10) being configured to make it possible to adjust the length of the emitting optical fibre and/or of the receiving optical fibre.

8. Device according to Claim 7, **characterized in that** it comprises a resin block positioned on the adapting element in order to fix the position of the emitting optical fibre and of the receiving optical fibre.

9. Device according to Claim 7 or 8, **characterized in that** the emitting optical fibre (F_E) and the receiving optical fibre (F_R) each comprise a distal end with respect to the light source (S) and to the photodetector (PH), respectively, and **in that** the distal end of the receiving optical fibre (F_R) is set back with respect to that of the emitting optical fibre (F_E).
